# EUROPÄISCHE PATENTANMELDUNG

(11) **EP 2 397 540 A1**
(43) Veröffentlichungstag der Anmeldung: **21.12.2011**
(21) Anmeldenummer: 10450103.6
(22) Anmeldetag: 18.06.2010
(51) Int. Cl.: C12M 1/107, F17C 1/00

(54) **Gasspeicher**

(71) Anmelder: Sattler AG, 8041 Graz-Thondorf (AT)
(72) Erfinder: Wiedau, Helmut, 48161 Münster (DE)
(74) Vertreter: Ellmeyer, Wolfgang

(57) **Zusammenfassung**

Gasspeicher (10) mit zumindest einer gasdichten Membran (1, 2), wobei in der zumindest einen gasdichten Membran (1, 2) zumindest jeweils eine Öffnung (4) ausgebildet ist, dass ein Bedien-Element (3) vorgesehen ist, dessen Umfang mit dem Rand der Membranöffnung(en) (4) gasdicht verbunden ist, wobei das Bedien-Element (3) zumindest eine Durchbrechung (6, 7, 70, 71, 72, 73, 74, 75, 76, 77) umfasst, die wahlweise gasdicht verschließbar ist oder sind, und wobei eine Halterung (15) für das Bedien-Element (3) vorgesehen ist, wobei die Lastabtragung über die Halterung (15) außerhalb des Gasspeichers (10) erfolgt.

## Beschreibung

Als Gasspeicher werden im Rahmen der Erfindung alle Behälter, ob rund eckig oder mit abgeböschten Innenwänden, z.B. Erdbeckenfermenter bezeichnet, die in der Lage sind, Gas zu speichern, unabhängig davon ob das zu speichernde Gas innerhalb dieses Behälters erzeugt oder von außen zugeleitet wird.

Bei der Erzeugung von Biogas, insbesondere bei der Fermentation, der Nachgärung und der Endlagerung spielt die Speicherung des Biogases eine bedeutende Rolle.

Besonders die Veränderbarkeit des Gasspeichervolumens zur Anpassung an die Schwankungen bei der Gaserzeugung stellt eine wichtige Anforderung dar, die durch Membran-Gasspeicher erfüllt wird. Eine Gasspeicherfolie bzw. -membran, sie kann aus verschiedenen Materialien bestehen, z.B. aus LDPE, PP, PP/PVC oder allgemein einem beschichteten Gewebe, wird durch Klemmung an einer Behälterwand eingespannt, welche den Innenraum umgibt, in dem die Fermentation z.B. von bei der Nutztierhaltung anfallenden Ausscheidungen durchgeführt wird. Das entstehende oder eingespeiste Biogas füllt den gasdichten Innenraum, sodass sich die Speichermembran entsprechend ausweitet.

Es kann auch eine zweischalige gasdichte Abdeckung, wie z.B. bei einem Doppelmembran-Biogasspeicher vorgesehen sein, bei der sich die Innenmembran je nach Gasvolumen hebt und senkt und von der sie umgebenden Außenmembran geschützt wird. Durch Veränderung des zwischen der Innen- und Außenmembran wirkenden Stützdruckes wird die Form der Außenmembran weitgehend konstant gehalten, während die Innenmembran entsprechend ihrer Befüllung sich ausdehnen oder zusammenziehen kann. Die Außenmembran nimmt sämtliche Belastungen durch Schnee, Wind und andere Witterungseinflüsse auf.

Die Innenmembran bildet somit die gasdichte Abdeckung des Gasspeichers. Der Druck im Stützluftraum zwischen der Innenmembran und der Außenmembran überträgt sich über die Innenmembran auf das gespeicherte Biogas. Je nach Füllstand schwebt daher die Innenmembran auf unterschiedlicher Höhe zwischen einem im Inneren des Gasspeichers angebrachten Gurtsystem und der Außenmembran.

Für die Zu- und Ableitung von Gas, Durchführungen jeglicher Art sowie für die Einbringung bzw. Lagerung von Rührwerken wird eine, insbesondere die Bodenabdeckung des Gasspeichers, möglichst schonende Form von Durchführungen angestrebt, um das Leckwerden des Gasspeichers zu verhindern.

Aufgabe der Erfindung ist es daher, einen Gasspeicher der eingangs genannten Art anzugeben, mit dem Durchführungen für Gas- und Flüssigkeitsrohre, Leitungen sowie die Einbringung und Lagerung von Rührwerken ohne Durchdringungen der Bodenabdeckung geschaffen werden und zugleich eine Beschädigung oder übergroße Belastung der Gasmembranen des Gasspeichers hintan gehalten wird.

Erfindungsgemäß wird dies dadurch erreicht, dass in der zumindest einen gasdichten Membran zumindest jeweils eine Öffnung ausgebildet ist, dass ein Bedien-Element vorgesehen ist, dessen Umfang mit dem Rand der Membranöffnung(en) gasdicht verbunden ist, wobei das Bedien-Element zumindest eine Durchbrechung umfasst, an die wahlweise zumindest ein Durchführungselement anschließbar ist oder die gasdicht verschließbar ist, und dass eine Halterung für das Bedien-Element vorgesehen ist, wobei die Lastabtragung über die Halterung außerhalb des Gasspeichers erfolgt.

Über das Bedien-Element können Durchführungen für diverse Rohrleitungen oder für Servicezwecke in vorgefertigter Weise in der gasdichten Membran eingesetzt werden, um die Gaszu- und ableitung sowie Substratzu- und abführung durch die gasdichte Membran hindurch zu bewerkstelligen. Weiters kann mittels des Bedien-Elements eine Lagerung eines Rührwerks erzielt werden. Zu diesem Zweck kann die zumindest eine Durchbrechung mit zumindest einer gasdichten Hindurchführung für ein Antriebselement versehen sein.

Durch die für das Bedien-Element vorgesehene separate Halterung können statische Kräfte auf die Behälterwände des Gasspeichers abgelastet werden. Dies ist besonders vorteilhaft bei statisch instabilen Behältern oder für Nachrüstungen von vorhandenen Behältern, für die statische Nachweise nicht mehr erbracht werden können. Alle vom Bedien-Element ausgehenden statischen Lasten werden über die Halterung nach außen abgelastet.

Das Bedien-Element kann durch eine vorzugsweise rechteckförmige Platte gebildet sein, die eine entsprechende Wandstärke aufweist, um die auf sie wirkenden Kräfte aufzunehmen, welche durch Rohrhindurchführungen oder Lagerung von Antriebsvorrichtungen während des Betriebs auftreten.

Bei höheren auf das Bedien-Element einwirkenden Belastungen kann gemäß einer weiteren Ausführungsform der Erfindung das Bedien-Element durch im Wesentlichen in einer Ebene angeordnete und miteinander verbundene Profilstäbe gebildet sein, die eine Abdeckung tragen, in welcher die zumindest eine Durchbrechung ausgebildet ist.

Die Herstellung einer Verbindung zwischen dem Öffnungsrand der gasdichten Membran und dem Bedien-Element kann gemäß einer Ausführungsform der Erfindung dadurch erfolgen, dass das Bedien-Element randseitig zumindest einen Klemmflanschring aufweist, an dem die zumindest eine gasdichte Membran gasdicht klemmbar ist. Auf diese Weise ist das Bedien-Element in der gasdichten Membran eingesetzt und stellt die Hindurchführungen für verschiedene Zwecke bzw. eine Lagerstelle für Antriebselemente bereit.

Die während des Betriebs auf das Bedien-Element einwirkenden Belastungen, die sich z.B. aus dem Gewicht der Rohrleitungen oder des gelagerten Antriebselements ergeben, werden über die Halterung zwar nach außen abgetragen, dennoch können durch das Bedien-Element zusätzliche Spannungen in der gasdichten Membran auftreten, die Walkbewegungen hervorrufen, welche negative Auswirkungen auf die Festigkeit der Membran haben können. Aus diesem Grund kann zwischen dem Bedien-Element und dem Öffnungsrand der gasdichten Membran eine Ausgleichsmembran angeordnet sein, welche die Auswirkungen der Walkbewegungen auf die gasdichte Membran herabsetzt.

Gemäß einer weiteren Ausführungsform der Erfindung weist das Bedien-Element randseitig einen inneren Klemmflanschring, an dem der Rand einer Öffnung der Ausgleichsmembran klemmbar ist, und einen äußeren Klemmflanschring auf, an dem der Außenrand der Ausgleichsmembran klemmbar ist und der einen größeren Umfang als jener des inneren Klemmflanschringes aufweist, wobei zusätzlich am äußeren Klemmflanschring der Öffnungsrand der zumindest einen gasdichten Membran klemmbar ist. Die Ausgleichsmembran ist über den inneren Klemmflanschring und den äußeren Klemmflanschring zwischen das Bedien-Element und den Öffnungsrand der gasdichten Membran gespannt.

In weiterer Ausbildung der Erfindung kann die zumindest eine Durchbrechung des Bedien-Elements durch einen Deckel mit einem Loch zur Hindurchführung eines Rührwerks-Antriebselements gasdicht verschließbar sein, wobei das Antriebselement des Rührwerks durch das Hindurchführungsloch dichtend hindurchgeführt ist und das Rührwerk am Bedien-Element festgelegt ist. Die Antriebswelle einer auf der Außenseite des Bedien-Elements angebrachten Antriebsvorrichtung ist somit gasdicht durch das Bedien-Element hindurchgeführt und ragt in das Innere des Gasspeichers, wobei die Antriebswelle von außen angetrieben werden kann.

Weiters kann vorgesehen sein, dass die zumindest eine Durchbrechung des Bedien-Elements eine Service-Öffnung umfasst, die durch einen Deckel mit zumindest einem Sichtglasbereich gasdicht verschließbar ist. Durch den Sichtglasbereich können von außen Beobachtungen im Inneren des erfindungsgemäßen Gasspeichers durchgeführt werden. Bei Abnahme des Deckels können Servicearbeiten durchgeführt werden.

Das Bedien-Element kann mit Rohr- oder Leitungshindurchführungen ausgestattet sein, um Zu- und Ableitung von Gasen und Flüssigkeiten zu erlauben. Deshalb kann in Weiterbildung der Erfindung die zumindest eine Durchbrechung des Bedien-Elements gasdichte Rohrdurchführungen für Rohre zur Zu- und Ableitung von Substrat und/oder gasdichte Rohrdurchführungen für Rohre zur Zu- und Ableitungen von Gas und/oder gasdichte Rohrdurchführungen für Heizungsrohre und/oder weitere gasdichte Durchführungen für Steuer-, Sensor- und Überwachungseinrichtungen umfassen, ohne dabei den Betrieb des Gasspeichers zu stören.

Einen wesentlichen Anteil an der Erfindung liefert die Halterung, mit deren Hilfe die durch das Bedien-Element hervorgerufenen statischen Kräfte nach außen abgetragen werden, um zu verhindern, dass die gasdichte Membran durch die am Bedien-Element auftretenden Kräfte beschädigt werden.

Die Halterung für das Bedien-Element kann z.B. durch eine außerhalb des Gasspeichers angeordnete Gerüst- oder Mastkonstruktion gebildet sein, die getrennt vom Gasspeicher errichtet ist, um keine Belastung an der Gasspeicherkonstruktion entstehen zu lassen.

Gemäß einem weiteren Ausführungsbeispiel der Erfindung kann die Gerüst- oder Mastkonstruktion durch einen freistehenden Mast gebildet sein, der an seiner Oberseite eine Auflagerung für zumindest eine Seite des Bedien-Elements aufweist.

Weiters kann der freistehende Mast an seiner Oberseite eine Standfläche aufweisen, wobei an den Seiten der Standfläche verstellbare Haltestäbe angebracht sind, welche eine Rückverankerung ausbilden und das Bedien-Element in einer im wesentlichen schrägen Stellung in einem einstellbaren Winkel gegenüber der Vertikalen halten. Die Schrägstellung des Bedien-Elements kann somit verändert werden und ist bevorzugt dem jeweiligen Böschungsinnenwinkel anzupassen.

Eine andere Variante des Halterungselements kann darin bestehen, dass die Halterung für das Bedien-Element durch eine außerhalb des Gasspeichers angeordnete Rahmenkonstruktion gebildet ist, die auf der Höhe der Beckenkrone des Gasspeichers auf einem Einzelfundament lagert. Auf diese Weise kann ein erfindungsgemäßes Bedien-Element auch für Erdbeckenfermenter zum Einsatz gelangen, wodurch Zu- und Ableitungen und sonstige Apparaturen durch die zumindest eine gasdichte Membran hindurchführbar sind.

Nachfolgend wird die Erfindung anhand der in den beigeschlossenen Zeichnungen dargestellten Ausführungsbeispiele eingehend erläutert. Es zeigt dabei
Fig. 1 eine teilweise Schrägansicht einer Ausführungsform des erfindungsgemäßen Gasspeichers;
Fig.2 eine Seitenansicht eines Rührwerks zur Montage im erfindungsgemäßen Gasspeicher gemäß Fig.1;
Fig.3 eine Draufsicht auf ein Bedien-Element des erfindungsgemäßen Gasspeichers gemäß Fig. 1;
Fig.4 eine Schrägansicht des Bedien-Elements gemäß Fig.3 mit Durchführungs-Abdeckung;
Fig.5 ein Detail einer schematischen Schnittdarstellung des Bedien-Elements einer weiteren Ausführungsform der Erfindung;
Fig.6 eine Draufsicht auf das Bedien-Element einer weiteren Ausführungsform der Erfindung;
Fig.7 einen Schnitt durch eine weitere Ausführungsform eines erfindungsgemäßen Gasspeichers;
Fig.8 eine Draufsicht auf die Ausführungsform gemäß Fig. 7;
Fig.9 eine vergrößerte Draufsicht auf ein Detail des Gasspeichers gemäß Fig.7;
Fig.10 einen Schnitt AA durch das Detail gemäß Fig.9 und
Fig.11 einen Schnitt BB durch das Detail gemäß Fig. 9.

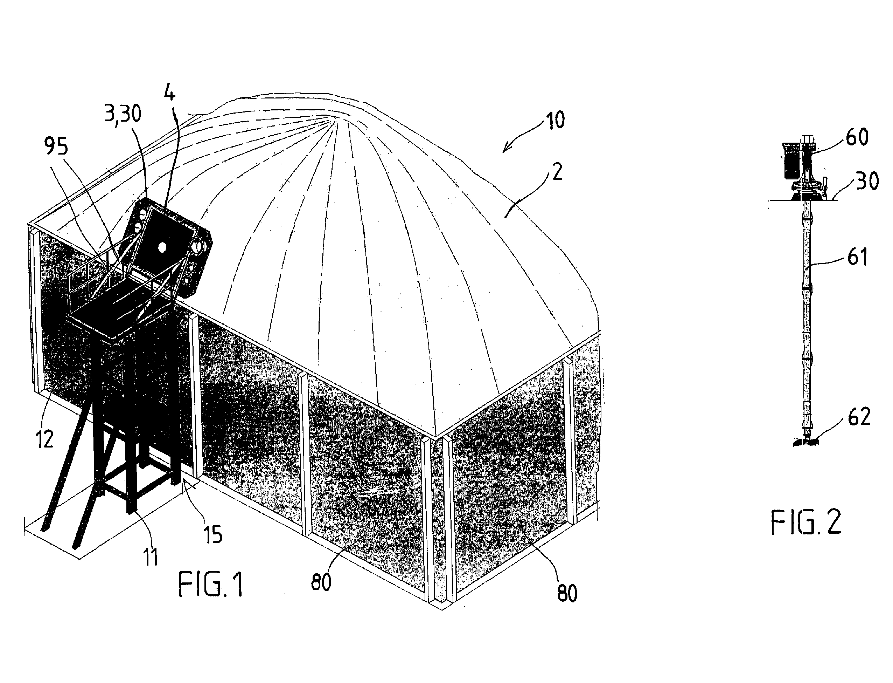

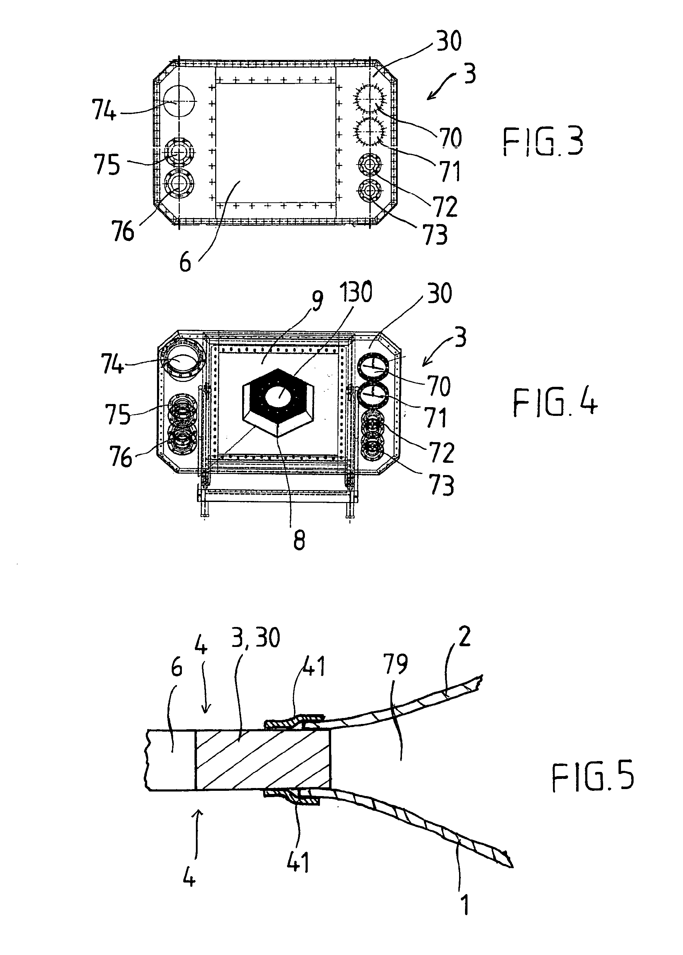

Fig.1 zeigt einen Gasspeicher 10 in Form eines Fermenters mit einer nicht dargestellten gasdichten Innenmembran 1 (Fig.5) und einer gasdichten Außenmembran 2, die beide mit ihren Rändern an der Oberseite von Seitenwänden 80 des Gasspeichers 10 gasdicht geklemmt sind. Es können auch nur eine oder mehr als zwei gasdichte Membranen vorgesehen sein.

Zwischen der Innenmembran 1 und der Außenmembran 2 ist in an sich bekannter Weise ein Stützgasraum 79 (Fig.5) ausgebildet, der z.B. durch Druckluft so geregelt wird, dass bei sich veränderndem Gasspeicherinhalt die Außenmembran 2 ihre Form unverändert beibehält, während im Inneren die Innenmembran 1 je nach Gasfüllstand sich senkt und hebt.

Erfindungsgemäß ist sowohl in der Innenmembran 1 als auch in der Außenmembran 2 jeweils eine Membranöffnung 4 ausgebildet und es ist ein Bedien-Element 3 vorgesehen, dessen Umfang mit dem Rand der Membranöffnungen 4 über Klemmflanschringe 41, 42 gasdicht verbunden ist. Das Bedien-Element 3 ist somit in den Öffnungen 4 der Innen- und Außenmembran 1, 2 eingelassen und dient der Hindurchführung von Rohren, Leitungen etc., der Lagerung einer Antriebsvorrichtung und Hindurchführung von Antriebselementen sowie dem verschließbaren Zugang in das Innere des Gasspeichers 10.

Das Bedien-Element 3 weist zu diesem Zweck mehrere Durchbrechungen 6, 70, 71, 72, 73, 74, 75, 76 (Fig.3) auf, an die wahlweise zumindest ein Durchführungselement anschließbar oder die gasdicht verschließbar sind.

Das Bedien-Element 3 ist durch eine Platte 30 gebildet, an deren Außenrand der Öffnungsrand der Innenmembran 1 und jener der Außenmembran 2 gasdicht festgeklemmt sind, wie dies in Fig. 5 vergrößert dargestellt ist.

Die zentral angeordnete rechteckförmige Durchbrechung 6 ist mit einem Durchführungselement in Form eines Deckels 9 mit einer gasdichten Hindurchführung 8 (Fig.4) für ein in Fig.2 dargestelltes Antriebselement 61 versehen.

Der Deckel 9 weist dazu ein Loch 130 zur Hindurchführung des Antriebselements 61 auf.

Das Bedien-Element 3 kann diesbezüglich für den Zugriff auf im Inneren des Gasspeichers 10 fix installierte Stabrührwerke oder für die Lagerung von Tauchrührgeräten oder auch Tauchpumpen ausgebildet sein.

Die kreisförmigen Durchbrechungen 70, 71, 72, 73, 74, 75, 76 dienen unterschiedlichen Zwecken und sind, z.B. zur Anbringung von Instrumenten, als gasdichte Rohrdurchführungen für Rohre zur Zu- und Ableitung von Substrat und/oder gasdichte Rohrdurchführungen für Rohre zur Zu- und Ableitung von Gas und/oder gasdichte Rohrdurchführungen für Heizungsrohre und/oder weitere gasdichte Durchführungen für Steuer-, Sensor- und Überwachungseinrichtungen verwendbar.

Die Abtragung der Last des Bedien-Elements 3 geschieht über eine Halterung 15 in Form einer Gerüst- oder Mastkonstruktion, die durch einen außerhalb des Gasspeichers 10 angeordneten, freistehenden Mast 11 gebildet ist, der an seiner Oberseite eine Auflagerung für eine Seite des Bedien-Elements 3 aufweist.

Weiters ist der Mast 11 an seiner Oberseite mit einer Standfläche 12 versehen, an deren Seiten verstellbare Haltestäbe 95 als Rückverankerung angebracht sind, welche die Platte 30 in einer im wesentlichen schrägen Stellung in einem einstellbaren Winkel gegenüber der Vertikalen hält. Die Schrägstellung ist dabei dem jeweiligen Böschungsinnenwinkel des Fermenters anzupassen.

Fig.2 zeigt ein auf der Platte 30 angebrachtes Rührwerk 60, dessen Welle 61 über die Hindurchführung 8 durch die Durchbrechung 6 hindurchgeführt ist und nach unten in den Fermenter ragt. Die Gasdichtheit der Hindurchführung 8 wird dabei z.B. mittels Simmeringen und Fettdichtungen erreicht.

Die Welle weist an ihrem freien Ende eine Rührschaufel 62 auf, mit der das während des Betriebs im Bodenbereich des Fermenters vorhandene Substrat gerührt werden kann.

Die in Fig. 2 gezeigte Anordnung kann in der Durchbrechung 6 des in Fig.1 gezeigten Bedien-Elements 3 eingesetzt und fixiert werden.

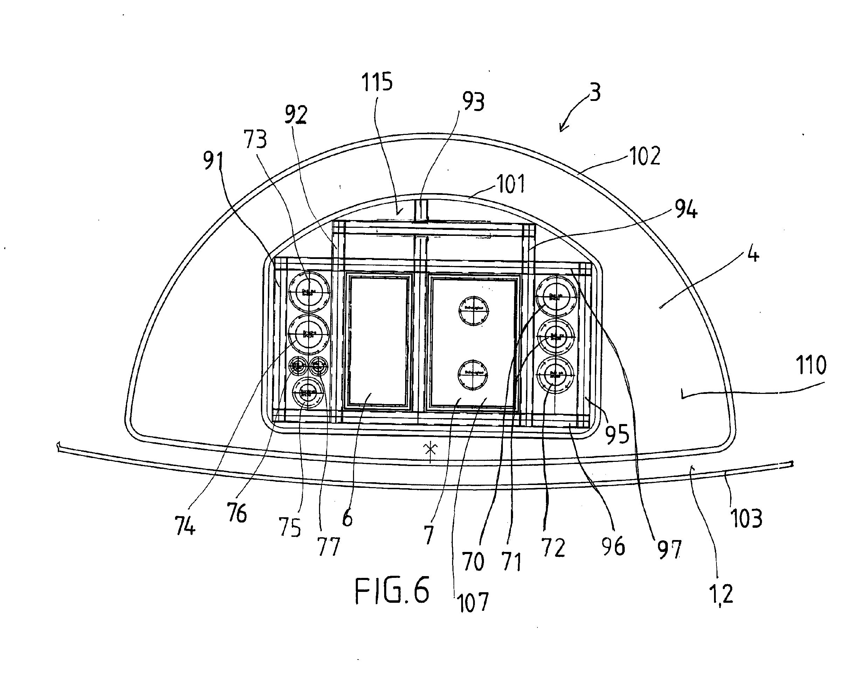

Fig.6 zeigt eine weitere Ausführungsform des Bedien-Elements 3, das durch im Wesentlichen in einer Ebene angeordnete und miteinander verbundene Quer-Profilstäbe 91, 92, 93, 94, 95 und Längs-Profilstäbe 96, 97 gebildet ist, die eine Abdeckung 115 tragen, in welcher die Durchbrechungen 6, 7, 70, 71, 72, 73, 74, 75, 76, 77 ausgebildet sind, an welche z.B. Rohrdurchführungen für die Zu- und Ableitung von Gas sowie Rohrdurchführungen für Heizungsrohre, Überwachungseinrichtungen, Steuerungen und Sensoren angeschlossen werden können.

Die zentral angeordneten, rechteckförmigen Durchbrechungen 6, 7 dienen einerseits zur Lagerung von Antriebsvorrichtungen und Hindurchführung von Antriebselementen aber können auch z.B. von mit Sichtfensterbereichen versehenen Deckeln verschlossen sein. Diese können als Serviceöffnung verwendet werden.

Das aus den Quer- und Längs-Profilstäben 91, 92, 93, 94, 95, 96, 97 sowie der Abdeckung 115 gebildete Bedien-Element 3 wird von einer Ausgleichsmembran 110 umgeben. Die Ausgleichsmembran 110 ist dazu so konfektioniert, dass sie eine Öffnung aufweist, deren Rand mit einem randseitig am Bedien-Element 3 vorgesehenen Klemmflanschring 101 geklemmt ist.

Weiters ist ein äußerer Klemmflanschring 102 ausgebildet, der einen größeren Umfang als der des inneren Klemmflanschrings 101 aufweist, an dem der Außenrand der Ausgleichsmembran 110 geklemmt ist. Zugleich ist der Öffnungsrand der Innenmembran 1 und der Außenmembran 2 am äußeren Klemmflanschring 102 geklemmt.

Die Innenmembran 1 und die Außenmembran 2 sind ihrerseits an ihren äußeren Rändern mit einem weiteren Klemmflanschring 103 an den Wänden des Gasspeichers 10 geklemmt.

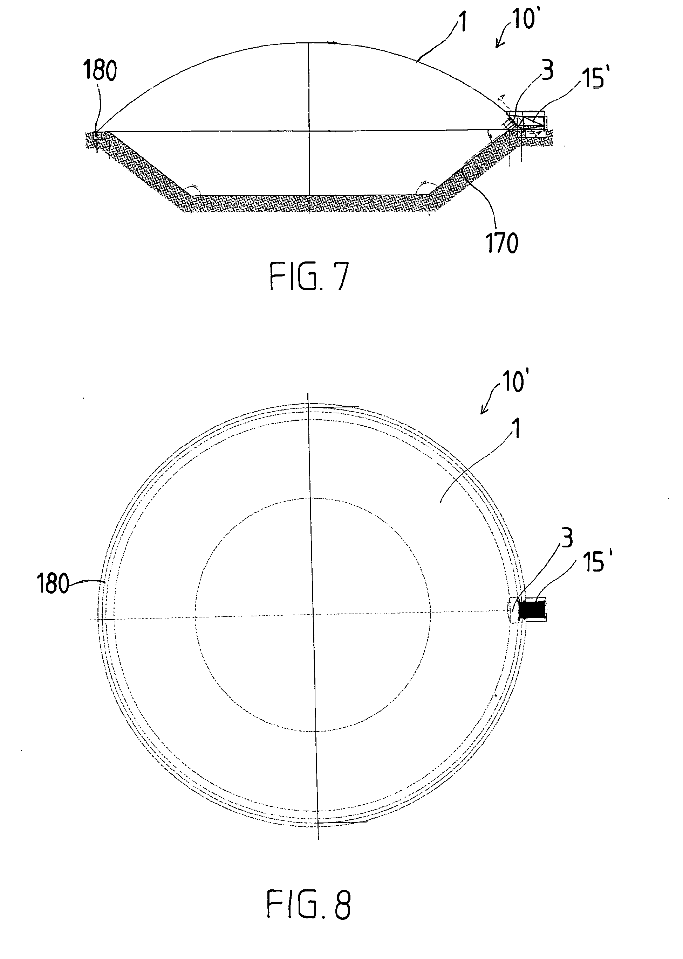

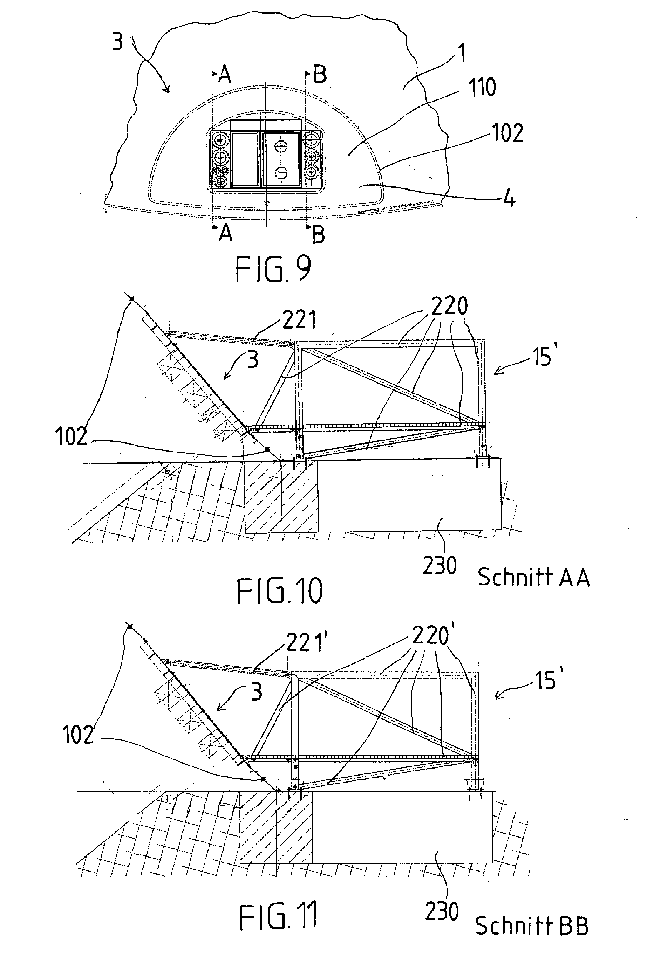

Fig.7 bis 11 zeigen einen weiteren erfindungsgemäßen Gasspeicher 10', der als Erdbeckenfermenter mit einem abgesenkten Boden 170 ausgebildet ist, der mit einer nicht dargestellten Abdeckfolie abgedeckt ist. Die einzige gasdichte Membran 1 ist randseitig entlang des Umfangs der Beckenkrone 180 gasdicht geklemmt und variiert je nach Füllstand in ihrer Scheitelpunkthöhe. Als Bedien-Element 3 kommt das in Fig.6 im Detail gezeigte Bedien-Element zum Einsatz.

Die Halterung für das Bedien-Element 3 ist durch eine außerhalb des Gasspeichers 10' angeordnete Rahmenkonstruktion 15' gebildet, die auf der Höhe der Beckenkrone 180 des Gasspeichers 10' auf einem Einzelfundament 230 lagert.

Die Rahmenkonstruktion 15' ist aus mehreren Stützen und Streben 220, 220' und aus zwei Auslegerarmen 221, 221' gebildet, über welche die Rückverankerung des Bedienelements 3 geschieht.

## Patentansprüche

1. Gasspeicher (10, 10') mit zumindest einer gasdichten Membran (1, 2), **dadurch gekennzeichnet, dass** in der zumindest einen gasdichten Membran (1, 2) zumindest jeweils eine Öffnung (4) ausgebildet ist, dass ein Bedien-Element (3) vorgesehen ist, dessen Umfang mit dem Rand der Membranöffnung(en) (4) gasdicht verbunden ist, wobei das Bedien-Element (3) zumindest eine Durchbrechung (6, 7, 70, 71, 72, 73, 74, 75, 76, 77) umfasst, an die wahlweise zumindest ein Durchführungselement (8) anschließbar ist oder die gasdicht verschließbar ist/sind, und dass eine Halterung (15, 15') für das Bedien-Element (3) vorgesehen ist, wobei die Lastabtragung über die Halterung (15, 15') außerhalb des Gasspeichers (10) erfolgt.

2. Gasspeicher nach Anspruch 1, **dadurch gekennzeichnet, dass** die zumindest eine Durchbrechung (6, 7, 70, 71, 72, 73, 74, 75, 76, 77) mit zumindest einer gasdichten Hindurchführung (8) für ein Antriebselement (61) versehen ist.

3. Gasspeicher nach Anspruch 1 oder 2, **dadurch gekennzeichnet, dass** das Bedien-Element (3) durch eine Platte (30) gebildet ist.

4. Gasspeicher nach Anspruch 1 oder 2, **dadurch gekennzeichnet, dass** das Bedien-Element (3) durch im Wesentlichen in einer Ebene angeordnete und miteinander verbundene Profilstäbe (91, 92, 93, 94, 95, 96, 97) gebildet ist, die eine Abdeckung (115) tragen, in welcher die zumindest eine Durchbrechung (6, 7, 70, 71, 72, 73, 74, 75, 76, 77) ausgebildet ist.

5. Gasspeicher nach einem der Ansprüche 1 bis 4, **dadurch gekennzeichnet, dass** das Bedien-Element (3) randseitig zumindest einen Klemmflanschring (41, 42) aufweist, an dem die zumindest eine gasdichte Membran (1, 2) gasdicht klemmbar ist.

6. Gasspeicher nach einem der Ansprüche 1 bis 5, **dadurch gekennzeichnet, dass** eine Ausgleichsmembran (110) vorgesehen ist, und dass das Bedien-Element (3) randseitig einen inneren Klemmflanschring (101), an dem der Rand einer Öffnung der Ausgleichsmembran (110) klemmbar ist, und einen äußeren Klemmflanschring (102) aufweist, an dem der Außenrand der Ausgleichsmembran (110) klemmbar ist und der einen größeren Umfang als jener des inneren Klemmflanschringes (101) aufweist, wobei zusätzlich am äußeren Klemmflanschring (102) der Öffnungsrand der zumindest einen gasdichten Membran (1, 2) klemmbar ist.

7. Gasspeicher nach einem der Ansprüche 1 bis 6, **dadurch gekennzeichnet, dass** die zumindest eine Durchbrechung (6) des Bedien-Elements (3) durch einen Deckel (9) mit einem Loch (130) zur Hindurchführung eines Rührwerks-Antriebselements (61) gasdicht verschließbar ist, wobei das Antriebselement (61) des Rührwerks (60) durch das Hindurchführungsloch (130) dichtend hindurchgeführt ist und das Rührwerk (60) am Bedien-Element (3) festgelegt ist.

8. Gasspeicher nach einem der Ansprüche 1 bis 7, **dadurch gekennzeichnet, dass** die zumindest eine Durchbrechung des Bedien-Elements (3) eine Service-Öffnung (7) umfasst, die durch einen Deckel (107) mit zumindest einem Sichtglasbereich gasdicht verschließbar ist.

9. Gasspeicher nach einem der Ansprüche 1 bis 8, **dadurch gekennzeichnet, dass** die zumindest eine Durchbrechung des Bedien-Elements (3) gasdichte Rohrdurchführungen für Rohre zur Zu- und Ableitung von Substrat und/oder gasdichte Rohrdurchführungen für Rohre zur Zu- und Ableitung von Gas und/oder gasdichte Rohrdurchführungen für Heizungsrohre und/oder weitere gasdichte Durchführungen für Steuer-, Sensor- und Überwachungseinrichtungen umfasst.

10. Gasspeicher nach einem der vorhergehenden Ansprüche, **dadurch gekennzeichnet, dass** die Halterung für das Bedien-Element (3) durch eine außerhalb des Gasspeichers (10) angeordnete Gerüst- oder Mastkonstruktion (15) gebildet ist.

11. Gasspeicher nach Anspruch 10, **dadurch gekennzeichnet, dass** die Gerüst- oder Mastkonstruktion (15) durch einen freistehenden Mast (11) gebildet ist, der an seiner Oberseite eine Auflagerung für zumindest eine Seite des Bedien-Elements (3) aufweist.

12. Gasspeicher nach Anspruch 11, **dadurch gekennzeichnet, dass** der freistehende Mast (11) an seiner Oberseite eine Standfläche (12) aufweist, und dass an den Seiten der Standfläche (12) verstellbare Haltestäbe (95) angebracht sind, welche das Bedien-Element (3) in einer im wesentlichen schrägen Stellung in einem einstellbaren Winkel gegenüber der Vertikalen hält.

13. Gasspeicher nach einem der vorhergehenden Ansprüche 9 bis 12, **dadurch gekennzeichnet, dass** die Halterung für das Bedien-Element (3) durch eine außerhalb des Gasspeichers (10') angeordnete Rahmenkonstruktion (15') gebildet ist, die auf der Höhe der Beckenkrone (180) des Gasspeichers (10') auf einem Einzelfundament (230) lagert.
